# EUROPEAN PATENT APPLICATION

(11) **EP 4 265 736 A1**
(43) Date of publication of application: **25.10.2023**
(21) Application number: 21911492.3
(22) Date of filing: 21.12.2021
(51) Int. Cl.: C12Q 1/6883, A23K 10/16, A01K 67/027

(54) **METHOD FOR DIAGNOSIS OF NEURODEGENERATIVE DISEASES BY USING HPMA**

(30) Priority: 21.12.2020 KR 20200180210
(71) Applicant: UNIVERSITY-INDUSTRY COOPERATION GROUP OF KYUNG HEE UNIVERSITY, Yongin-si, Gyeonggi-do 17104 (KR)
(72) Inventor: OH, Myung Sook, Seoul 06291 (KR); KIM, Dong Hyun, Seoul 02823 (KR); CHOI, Jin Gyu, Bucheon-si, Gyeonggi-do 14694 (KR); HUH, Eugene, Seoul 04202 (KR)
(74) Representative: Callaghan, Dayle Anne
(86) International application number: PCT/KR2021/019539
(87) International publication number: WO 2022/139425

(57) **Abstract**

The present invention relates to a composition for the diagnosis of a degenerative brain disease, comprising hpmA, which is a substance derived from Proteus, Shigella, Klebsiella pneumoniae, or Citrobacter, preferably a *Proteus mirabilis* strain, from a biological sample of a subject. In addition, the present invention relates to a method for providing information for the diagnosis of a degenerative brain disease such as Parkinson's disease, brain neuritis (neuroinflammation), or Alzheimer's disease, by measuring the amount of hpmA.

## Description

### Technical Field

The present invention relates to a composition for the diagnosis of a degenerative brain disease, comprising hpmA from a biological sample of a subject. In addition, the present invention relates to a method for the diagnosis of a degenerative brain disease such as Parkinson's disease, brain neuritis (neuroinflammation), or Alzheimer's disease by measuring the amount of hpmA.

### Background Art

With the rapid increase in the elderly population, the number of patients with various neurodegenerative brain diseases such as Parkinson's disease, brain neuritis (neuroinflammation), or Alzheimer's disease is increasing, and interest in preventing and treating them is growing. Neurodegenerative brain disease is a disease that causes various symptoms such as motor disorder, memory disorder, and cognitive disorder due to degenerative changes in nerves, and the pathogenesis has not been completely identified to date.

Among these neurodegenerative brain diseases, Parkinson's disease is accompanied by symptoms such as tremor, bradykinesia, muscle rigidity, postural instability, and akinesia, and is known as a disease caused by cell death in dopaminergic nerve containing melanin pigment in the substantia nigra pars compacta (SNpc) of the brain and a deficiency of dopamine, a neurotransmitter in the corpus striatum, through various stimuli such as excessive production of reactive oxygen species, oxidative stress, misfolded proteins, and impaired mitochondrial function.

Dopamine is produced in the substantia nigra of the brain, and the nerve cells in the substantia nigra are intricately connected to the motor cortex and other several parts of the brain, and are connected to a region called the basal ganglia that enables the human body to perform movements smoothly and accurately, and dopamine regulates the function of the basal ganglia.

Until now, a method for treating Parkinson's disease includes drug therapy, surgical therapy, and physical therapy, and has mainly used a method of supplementing dopamine by administering L-dopa, a precursor of dopamine, but it is known in many clinical studies that continuous use of L-dopa causes serious side effects.

In addition, at the time when the main symptoms of Parkinson's disease are first expressed and diagnosed, more than 70% of dopaminergic neurons in the substantia nigra have already been destroyed, and the already dead neurons could not be revived. For this reason, these drugs aim only to improve symptoms. Therefore, there were limitations in early diagnosis, prevention, and treatment of Parkinson's disease.

A method for the diagnosis of Parkinson's disease include a method of imaging the substantia nigra or corpus striatum region of the brain using a method such as brain magnetic resonance image (MRI) examination, positron emission tomography (PET), single photon emission computed tomography (SPECT), tests for internal diseases (blood test, urine test, electrocardiogram, chest X-ray test) and the like, or a method of directly collecting brain tissue and examining a biological marker.

However, the diagnostic methods are expensive, have complex diagnostic procedures, and have problems in that the results obtained are inaccurate or that brain tissue must be directly collected, causing pain and burden to the patient, and the development of the Parkinson's disease diagnosis market is also insignificant.

Therefore, in order to improve therapeutic effects through early detection of Parkinson's disease and the like, there is an urgent need to develop a technology capable of rapidly diagnosing a neurodegenerative brain disease such as Parkinson's disease.

On the other hand, prolongation of gastrointestinal emptying time or constipation is a symptom experienced by most patients in the early stage of Parkinson's disease, and studies between Parkinson's disease and intestinal function can provide useful information for the development of a technology of diagnosing Parkinson's disease.

It is known that gut microbes maintain gut homeostasis and are involved in the production of neurotransmitters in the brain. It has also been found that changes in the gut microbes are associated not only with metabolic diseases such as obesity and diabetes, but also with mental diseases such as depression and autism, and degenerative brain diseases such as Alzheimer's disease and Parkinson's disease.

Strains such as Proteus, Shigella, Klebsiella pneumoniae, and Citrobacter are known to produce and secrete virulence factors of hpmA, but there is no known method for the diagnosis of a degenerative brain disease using hpmA. Among these, especially among the gut microbes, the *Proteus mirabilis* strain is a gram negative rod bacterium belonging to the family *Enterobacteriaceae,* and mainly lives in the human gastrointestinal tract, and acts as a pathogen when it enters the urinary tract, wound, or lung. It mainly causes infections in the urinary system, creates kidney stones or ureteral stones, and produces and secretes hpmA.

Accordingly, in order to solve the above conventional problems, the present inventors have studied the relationship between Parkinson's disease and gut microbes. As a result, the present inventors found for the first time that hpmA among virulence factors derived from gut microbes plays a key role in the induction of Parkinson's disease. Based on the above, the present inventors completed the present invention.

### Prior Art Document

### Patent Document

(Patent Document 1) Korean Patent Registration No. 10-1183620
(Patent Document 2) Korean Patent Registration No. 10-1081358
(Patent Document 3) Korean Patent Registration No. 10-1860566

### Detailed Description of Invention

### Technical Problem

An object of the present invention is to provide a composition for the diagnosis of a degenerative brain disease, comprising hpmA.

Another object of the present invention is to provide a method for the diagnosis of a degenerative brain disease, comprising the step of measuring the amount of hpmA from a biological sample of a subject.

Another object of the present invention is to provide a composition for the preparation of an animal model of a degenerative brain disease, comprising a strain comprising an hpmA gene as an active ingredient.

Another object of the present invention is to provide a method for the preparation of an animal model of a degenerative brain disease, comprising the step of administering a strain comprising an hpmA gene; and an animal model of a degenerative brain disease prepared by the preparation method.

Another object of the present invention is to provide a method for screening a therapeutic agent for a degenerative brain disease, comprising the step of administering a therapeutic candidate drug; the step of measuring the amount of hpmA from a biological sample of a subject; and the step of determining the therapeutic effect of a candidate drug.

### Solution to Problem

In order to achieve the objects of the present invention, the present invention will be described in detail below.

The present invention relates to a composition for the diagnosis of a degenerative brain disease, comprising hpmA.

As used herein, the term "degenerative brain disease" refers to a disease that shows degenerative changes in nerve cells of the central nervous system and causes various symptoms. Specifically, the degenerative brain disease may be Alzheimer's disease, Parkinson's disease, dementia, multiple sclerosis, Huntington's disease, amyotrophic lateral sclerosis, brain neuritis (neuroinflammation), multiple system atrophy, or Pick's disease.

As used herein, the term "diagnosis" means to confirm the presence or characteristics of a pathological condition, and in the present invention means to determine whether or not a degenerative brain disease such as Parkinson's disease, brain neuritis (neuroinflammation), or Alzheimer's disease has developed, but is not limited to diagnosis of the above diseases.

The present invention relates to a composition for the diagnosis of one or more degenerative brain diseases selected from the group consisting of Alzheimer's disease, Parkinson's disease, dementia, multiple sclerosis, Huntington's disease, amyotrophic lateral sclerosis, brain neuritis (neuroinflammation), multiple system atrophy and Pick's disease, and preferably to a composition for the diagnosis of Parkinson's disease.

The diagnostic composition of the present invention may comprise hpmA derived from Proteus, Shigella, Klebsiella pneumoniae, or Citrobacter, but is not limited thereto.

Preferably, the diagnostic composition of the present invention may comprise hpmA derived from a *Proteus mirabilis* strain.

The *Proteus mirabilis* strain of the present invention is a gram negative rod bacterium belonging to the family *Enterobacteriaceae,* and is a strain that biosynthesizes lipopolysaccharide (LPS) that causes inflammation, and is the second most common pathogen causing urinary tract infection in humans after *Escherichia coli. Proteus mirabilis* is distributed in various environments, and it is common to be infected through the urinary tract of humans or animals, but it can also be infected through the respiratory tract, skin, or intestinal system.

In particular, the *Proteus mirabilis* strain is known to produce and secrete various virulence factors such as hpmA, ureC, and mrpA. Among them, hpmAis a hemolysin produced by the *Proteus mirabilis* strain and is one of the endotoxins.

The present invention relates to a composition for the diagnosis of Parkinson's disease, comprising hpmA derived from a *Proteus mirabilis* strain.

The present invention provides a method for the diagnosis of a degenerative brain disease, comprising the step of measuring the amount of hpmA from a biological sample of a subj ect.

In a specific embodiment of the present invention, the subject may be an animal including a human.

The hpmA of the present invention may be derived from Proteus, Shigella, Klebsiella pneumoniae, or Citrobacter. Preferably, the hpmA of the present invention may be derived from a *Proteus mirabilis* strain, but is not limited thereto.

The present invention provides a method for the diagnosis of a degenerative brain disease, comprising the step of measuring the amount of hpmA from a biological sample of a subject, and the step of comparing the amount of hpmA measured from a biological sample of a subject with the amount of hpmA measured from a biological sample of a normal control group.

In addition, the present invention provides a method for the diagnosis of a degenerative brain disease, comprising the step of measuring the amount of hpmA from a biological sample of a subject, the step of comparing the amount of hpmA measured from a biological sample of a subject with the amount of hpmA measured from a biological sample of a normal control group, and the step of classifying the subject as having a disease or having a high possibility of developing a disease when the amount of hpmA measured from a biological sample of a subject is greater than the amount of hpmA measured from a biological sample of a normal control group.

As used herein, the term "biological sample" may be isolated from a living body, and includes a sample such as tissue, blood, whole blood, serum, plasma, saliva, sputum, cerebrospinal fluid, urine, large intestine tissue or feces of a subject, but is not limited thereto. The biological sample may be selected according to the patient's pathological condition and may be obtained by a method that does not harm the subject.

In the present invention, the normal control group may be an animal, including a human, not having a degenerative brain disease, and the normal control group that does not suffer from such a disease corresponds to a subject for comparison having the disease. Specifically, the normal control group is a subject that does not suffer from Parkinson's disease, and means an organism that does not suffer from the disease used for testing, examination, analysis, evaluation, and the like. Preferably, it may be a mammal, such as a human, mice, monkey, cow, horse, rabbit, dog, cat, sheep, and goat, that does not suffer from Parkinson's disease, and more preferably a human.

The present invention provides a composition for the preparation of an animal model of a degenerative brain disease, comprising a strain comprising an hpmA gene as an active ingredient, and preferably, a composition for the preparation of an animal model of Parkinson's disease.

As used herein, the term "animal model of Parkinson's disease" refers to a Parkinson's disease laboratory animal (excluding a human) constructed to study Parkinson's disease. In general, an animal model of Parkinson's disease is constructed by administering MPTP (1-methyl-4-phenyl-1,2,4,6-tetrahydropyridine), 6-OHDA (6-hydroxydopamine), rotenone and the like to a laboratory animal such as rodents or primates.

The strain of the composition for the preparation of an animal model of Parkinson's disease of the present invention may be *E. coli,* but is not limited thereto.

The present invention provides a method for the preparation of an animal model of a degenerative brain disease, comprising the step of administering a strain comprising an hpmA gene to an animal excluding a human; and an animal model of a degenerative brain disease prepared by the preparation method.

In the animal model, the animal includes a mice, monkey, cow, horse, rabbit, dog, cat, sheep, goat, and the like, excluding a human.

The present invention provides a method for screening a therapeutic agent for a degenerative brain disease, comprising the step of administering a therapeutic candidate drug; the step of measuring the amount of hpmA from a biological sample of a subject; and the step of determining the therapeutic effect of a candidate drug.

The method for screening a therapeutic agent for Parkinson's disease of the present invention may further comprise the step of measuring and comparing the amounts of hpmA before and after administration of the candidate drug.

As used herein, the term "therapeutic candidate drug" includes known compounds or newly synthesized compounds, natural products, biological agents, stem cells, and the like, and includes, without limitation, substances that are effective or expected to exhibit an effect in preventing or treating Parkinson's disease, and can be appropriately selected by those of ordinary skill in the art.

### Effects of Invention

In the present invention, it was experimentally confirmed that hpmA is directly related to motor ability and changes in dopaminergic neurons, and thus, the composition comprising hpmA of the present invention can be used for diagnosing a degenerative brain disease such as Parkinson's disease, brain neuritis (neuroinflammation), or Alzheimer's disease.

In addition, a degenerative brain disease can be diagnosed in a very simple and rapid manner by measuring the amount of hpmA from a biological sample of a subj ect and comparing it with the amount of hpmA in a normal control group.

In addition, an animal model of Parkinson's disease can be prepared by administering a composition comprising the strain comprising hpmA of the present invention as an active ingredient to an animal excluding a human, and can be usefully used in a method for screening a therapeutic agent for Parkinson's disease.

Therefore, it can rapidly diagnose a disease such as Parkinson's disease at an early stage compared to existing methods for the diagnosis of Parkinson's disease, thereby helping to improve the disease in patients with Parkinson's disease, and it can contribute to the development of new drugs with excellent effects by using it for screening of a therapeutic agent for Parkinson's disease.

### Brief Description of Drawings

Fig. 1 is a graph showing the results of measuring the hpmA content in feces in a normal group, a group administered with an EC strain, a group administered with an hpmA EC strain, and a group administered with a PM strain.
Fig. 2 is a graph showing the results of measuring voluntary locomotor activity in order to confirm whether a behavioral disorder is induced in a normal group, a group administered with an EC strain, a group administered with an hpmA EC strain, and a group administered with a PM strain.
Fig. 3 is a graph showing the results of measuring the amount of TH expression (optical density) in the striatum (ST) in order to confirm the change in dopaminergic neurons in a normal group, a group administered with an EC strain, a group administered with an hpmAEC strain, and a group administered with a PM strain.
Fig. 4 is a graph showing the results of measuring neuroglial cells (Iba-1 positive cells) in the substantia nigra pars compacta (SNpc) in order to confirm the induction of neuroinflammation in a normal group, a group administered with an EC strain, a group administered with an hpmAEC strain, and a group administered with a PM strain.
Fig. 5 is a graph showing the results of measuring voluntary locomotor activity in order to confirm whether a behavioral disorder is induced in a normal group, a group administered with a PM strain, and a group administered with an hpmA-/- PM strain.
Fig. 6 is a graph showing the results of measuring the amount of TH expression (optical density) in the striatum (ST) in order to confirm the change in dopaminergic neurons in a normal group, a group administered with a PM strain, and a group administered with an hpmA-/- PM strain.
Fig. 7 is a graph showing the results of measuring neuroglial cells (Iba-1 positive cells) in the substantia nigra pars compacta (SNpc) in order to confirm the induction of neuroinflammation in a normal group, a group administered with a PM strain, and a group administered with an hpmA-/- PM strain.
Fig. 8 is a graph showing the results of measuring the amount of α-synuclein aggregates (α-syn filaments) in the substantia nigra pars compacta (SNpc) in order to confirm whether abnormal protein hyperaggregation in the brain was induced in a normal group, a group administered with a PM strain, and a group administered with an hpmA-/- PM strain.
Fig. 9 is a graph showing the results of measuring the hpmA content in feces in a normal group and a group administered with MPTP/p.

### Best Mode for Carrying out the Invention

Hereinafter, with reference to the accompanying drawings, embodiments and examples of the present invention will be described in detail so that those of ordinary skill in the art to which the present invention belongs can easily carry out. However, the present invention may be implemented in various forms and is not limited to the embodiments and examples described herein.

Throughout the present specification, when a certain part "includes" a certain component, it means that other components may be further included, rather than excluding other components, unless otherwise stated.

Hereinafter, the present invention will be described in more detail through the examples, but the following examples are for illustrative purposes only and are not intended to limit the scope of the present invention.

### [Example 1]

### Construction of hpmA EC strain

The hpmA265 sequence, known as the active form of hpmA, was acquired in the form of genomic DNA, inserted into the pGEM-T vector, and cultured through transformation. Thereafter, the hpmA gene was extracted from the pGEM-T vector, and then ligated to the pET24a vector using a restriction enzyme, and then inserted into *Escherichia coli* (strain BL21(DE3)), and cultured. At this time, in order to improve the solubility of the prepared strain, after confirming the expression of hpmA by adding ubiquitin, the expressed strain was used.

### Experimental group

As shown in Table 1 below, PBS, *E. coli,* the prepared hpmA gene cloned *E. coli,* and *Proteus mirabilis* were orally administered to mice once a day for 5 days, respectively, and experiments were performed on a total of 4 groups.

**[Table 1]**

| | Group | Administration method |
|---|---|---|
| 1 | Normal | administration of PBS |
| 2 | WT EC | administration of *E. coli* strain (EC) |
| 3 | hpmA EC | administration of hpmA EC strain |
| 4 | WT PM | administration of *Proteus mirabilis* strain (PM) |

### [Example 2]

### Experiment measuring amount of hpmA expression

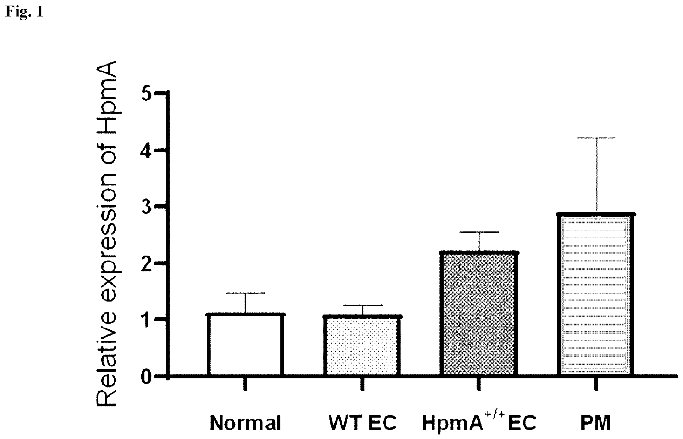

An experiment was performed to measure the amount of hpmA expression through real time polymerase chain reaction (real time-qPCR) using DNA extracted from the feces of the four groups of mice in Example 1 above and primers prepared from the hpmA sequence, and the results are shown in Table 2 below and Fig. 1. The corresponding result was calculated in terms of 16S and compared with the control group.

**[Table 2]**

| | Group | Expression amount (%) |
|---|---|---|
| 1 | Normal | 100±24.11 |
| 2 | WT EC | 97.59±14.38 |
| 3 | hpmA EC | 197.40±29.69 |
| 4 | WTPM | 258.39±104.09 |

As shown in Table 2 above and Fig. 1, it was found that the amount of hpmA expression in a group administered with an EC strain was similar to that of the control group, whereas the amounts of hpmA expression in a group administered with an hpmA EC strain and a group administered with a PM strain were increased by 2 times and by 2.5 times, respectively.

### [Example 3]

### Evaluation of behavior: Experiment measuring voluntary locomotor activity (Locomotor activity test)

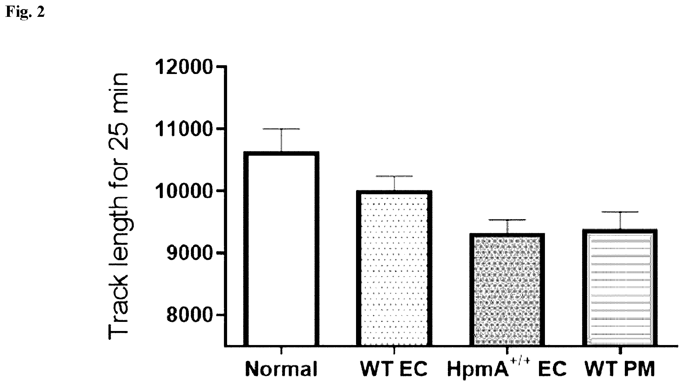

The evaluation of motor disorder in mice was performed through an experiment measuring voluntary locomotor activity (Locomotor activity test). The four groups of mice in Example 1 above were placed inside a 40X25X18 cm box, and their movements were tracked for 25 minutes, and the total moved distance (total length) was measured to evaluate whether or not the mice had a motor disorder, and the results are shown in Table 3 below and Fig. 2.

**[Table 3]**

| | Group | Moved distance (%) |
|---|---|---|
| 1 | Normal | 100±3.43 |
| 2 | WT EC | 94.14±2.12 |
| 3 | hpmA EC | 87.71±1.92 |
| 4 | WTPM | 88.28±2.59 |

As shown in Table 3 above and Fig. 2, it was found that the moved distance in a group administered with an EC strain was similar to that of the control group, whereas the moved distance in a group administered with an hpmAEC strain and a group administered with a PM strain was decreased. Therefore, it can be seen that hpmAhas a negative effect on motor ability.

### [Example 4]

### Histological analysis: Confirmation of change in dopaminergic neurons in striatum of brain

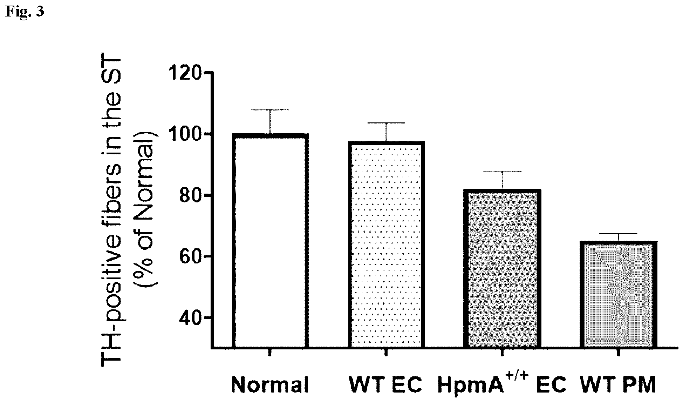

For the four groups of mice in Example 1 above, the amount of TH expression (optical density of TH) in the striatum (ST) region was measured by the brightness of the color of the colored region through immunochemical staining, and the results are shown in Table 4 below and Fig. 3.

**[Table 4]**

| | Group | Amount of TH expression (%) |
|---|---|---|
| 1 | Normal | 100±7.98 |
| 2 | WT EC | 97.74±7.97 |
| 3 | hpmA EC | 82.17±5.58 |
| 4 | WT PM | 65.22±5.97 |

As shown in Table 4 above and Fig. 3, it was found that the amount of TH expression in a group administered with an EC strain was similar to that of the control group, whereas the amount of TH expression in a group administered with an hpmA EC strain and a group administered with a PM strain was decreased. Therefore, it can be seen that hpmA induces the death of dopaminergic neurons in the brain.

### [Example 5]

### Histological analysis: Confirmation of induction of neuroinflammation in substantia nigra of brain

In order to confirm the induction of neuroinflammation in the substantia nigra pars compacta (SNpc) region, the region was defined through staining of TH positive cells, and neuroglial cells (Iba-1 positive cells) were measured, and then it was measured in terms of volume in consideration of the thickness of the tissue.

**[Table 5]**

| | Group | Neuroglial cells (%) |
|---|---|---|
| 1 | Normal | 100±17.70 |
| 2 | WT EC | 152.29±17.54 |
| 3 | hpmA EC | 194.64±24.81 |
| 4 | WT PM | 196.88±34.10 |

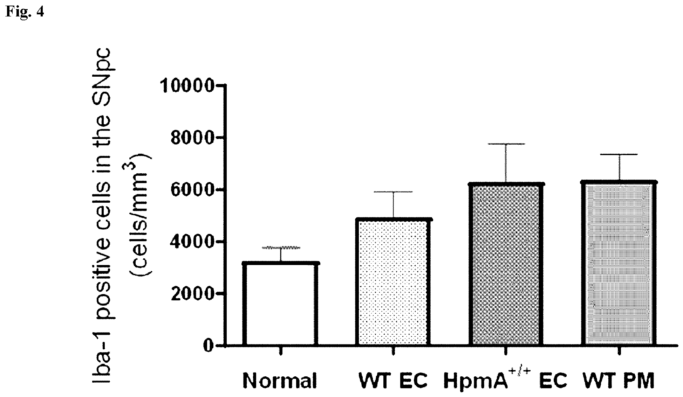

As shown in Table 5 and Fig. 4, it was found that the number of neuroglial cells in a group administered with an hpmA EC strain and a group administered with a PM strain was increased by 2 times compared to that of the control group. Therefore, it can be seen that hpmA induces neuroinflammation in the brain.

### [Example 6]

### Construction of hpmA-/- PM strain

The hpmA-/- PM strain was constructed using the TargeTron mutagenesis kit. The plasmid pAR1219, including the pBR3220 based vector used in the TargeTron mutagenesis kit, has the characteristic of suppressing the expression of the corresponding protein by inhibiting the transcription of a gene at a specific location. Resistance to a specific antibiotic inserted into the plasmid was induced, and the mutant strain was selectively cultured and used in the experiment.

### Experimental group

PBS, a WT PM strain, and a hpmA-/- PM strain were orally administered to mice once a day for 5 days, respectively, and experiments were performed on motor ability, dopaminergic neurons in the substantia nigra of the brain, neuroglial cells, and change in α-synuclein aggregates in the substantia nigra of the brain.

**[Table 6]**

| | Group | Administration method |
|---|---|---|
| 1 | Normal | administration of PBS |
| 2 | WT PM | administration of *Proteus mirabilis* strain (PM) |
| 3 | hpmA-/- PM | administration of hpmA mutant strain (hpmA-/-) |

### [Example 7]

### Evaluation of behavior: Experiment measuring voluntary locomotor activity (Locomotor activity test)

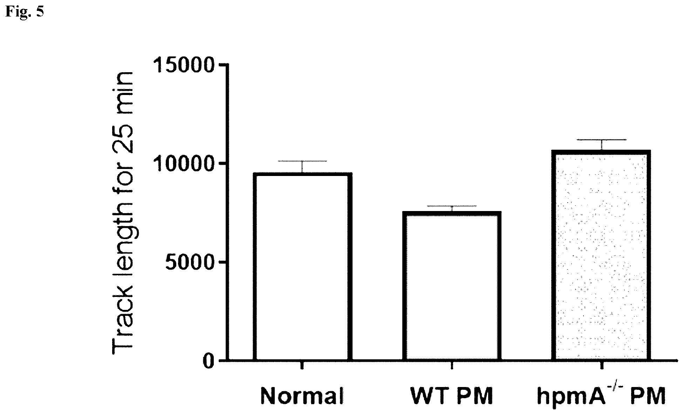

The evaluation of motor disorder in mice was performed through an experiment measuring voluntary locomotor activity (Locomotor activity test). The three groups of mice in Example 6 above were placed inside a 40X25X18 cm box, and their movements were tracked for 25 minutes, and the total moved distance (total length) was measured to evaluate whether or not there was a motor disorder, and the results are shown in Table 7 below and Fig. 5.

**[Table 7]**

| | Group | Moved distance (%) |
|---|---|---|
| 1 | Normal | 100±5.94 |
| 2 | WT PM | 79.32±2.44 |
| 3 | hpmA-/- PM | 111.65±5.04 |

As shown in Table 7 above and Fig. 5, it was found that the moved distance in a group administered with a PM strain was decreased compared to that of the control group, whereas the moved distance in a group administered with an hpmA-/- PM strain was similar to that of the control group. Therefore, it can be seen that hpmA has a negative effect on motor ability.

### [Example 8]

### Histological analysis: Confirmation of change in dopaminergic neurons in striatum of brain

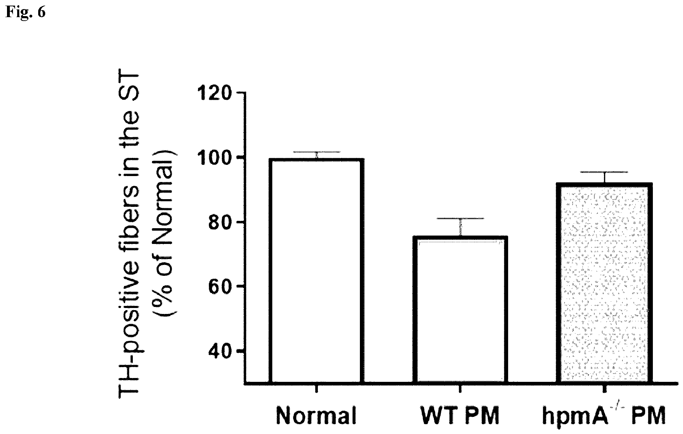

The amount of TH expression (optical density of TH) in the striatum (ST) region was measured by the brightness of the color of the colored region through immunochemical staining, and the results are shown in Table 8 below and Fig. 6.

**[Table 8]**

| | Group | Amount of TH expression (%) |
|---|---|---|
| 1 | Normal | 100±1.60 |
| 2 | WT PM | 75.97±4.51 |
| 3 | hpmA-/- PM | 92.29±3.23 |

As shown in Table 8 above and Fig. 6, it was found that the amount of TH expression in a group administered with a PM strain was decreased compared to that of the control group, whereas the amount of TH expression in a group administered with an hpmA-/- PM strain was similar to that of the control group. Therefore, it can be seen that hpmA is a factor that influences the strain-induced death of dopaminergic neurons in the brain.

### [Example 9]

### Histological analysis: Confirmation of induction of neuroinflammation in substantia nigra of brain

In order to confirm the induction of neuroinflammation in the substantia nigra pars compacta (SNpc) region, the region was defined through staining of TH positive cells, and neuroglial cells (Iba-1 positive cells) were measured, and then it was measured in terms of volume in consideration of the thickness of the tissue.

**[Table 9]**

| | Group | Neuroglial cells (%) |
|---|---|---|
| 1 | Normal | 100±17.02 |
| 2 | WT PM | 222.15±20.90 |
| 3 | hpmA-/- PM | 121.54±23.87 % |

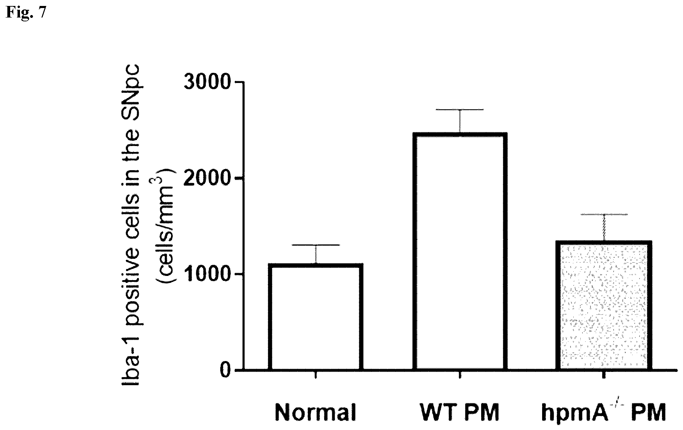

As shown in Table 9 and Fig. 7, it was found that the number of neuroglial cells in a group administered with a PM strain was increased compared to that of the control group, whereas the number of neuroglial cells in a group administered with an hpmA-/- PM strain was similar to that of the control group. Therefore, it can be seen that hpmA is a factor that influences the strain-induced neuroinflammation in the brain.

### [Example 10]

### Histological analysis: Confirmation of induction of α-synuclein aggregation in substantia nigra of brain

In order to confirm the induction of α-synuclein aggregation in the substantia nigra pars compacta (SNpc) region, the region was defined through staining of TH positive cells, and the aggregated α-synuclein (α-synuclein filaments) was measured, and then it was measured in terms of volume in consideration of the thickness of the tissue.

**[Table 10]**

| | Group | Aggregated α-synuclein (%) |
|---|---|---|
| 1 | Normal | 100±8.83 |
| 2 | WTPM | 388.23±35.38 |
| 3 | hpmA-/- PM | 127.92±13.89 |

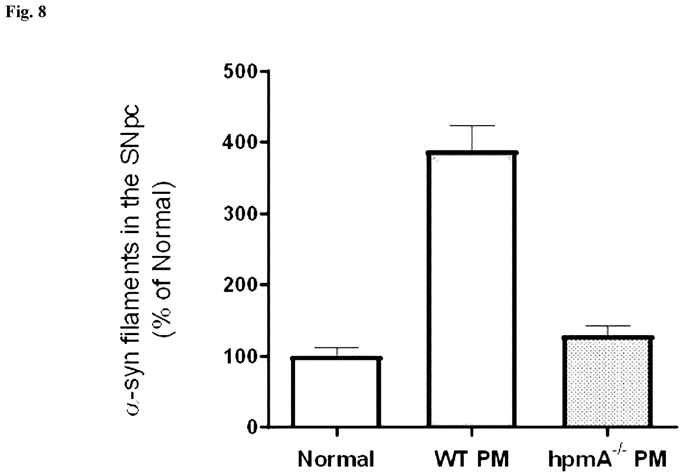

As shown in Table 10 and Fig. 8, it was found that the aggregated α-synuclein in a group administered with a PM strain was increased by more than 3 times compared to that of the control group, whereas the aggregated α-synuclein in a group administered with an hpmA-/- PM strain was similar to that of the control group. Therefore, it can be seen that hpmA is a factor that influences the strain-induced α-synuclein aggregation in the brain.

### [Example 11]

### Construction of mouse model of MPTP/p-induced Parkinson's disease at early stage

In order to construct a model of Parkinson's disease at early stage, 1-methyl-4-phenyl-1,2,3,6-tetrahydro-pyridine hydrochloride (MPTP) and probenecid (MPTP/p) were administered three times to mice twice a week, respectively. The corresponding mouse does not exhibit motor disorder, but exhibits non-motor symptoms (constipation, olfactory disorder, depression, etc.), and thus, is used as an animal model of Parkinson's disease at early stage.

### [Example 12]

### Measurement of amount of hpmA expression

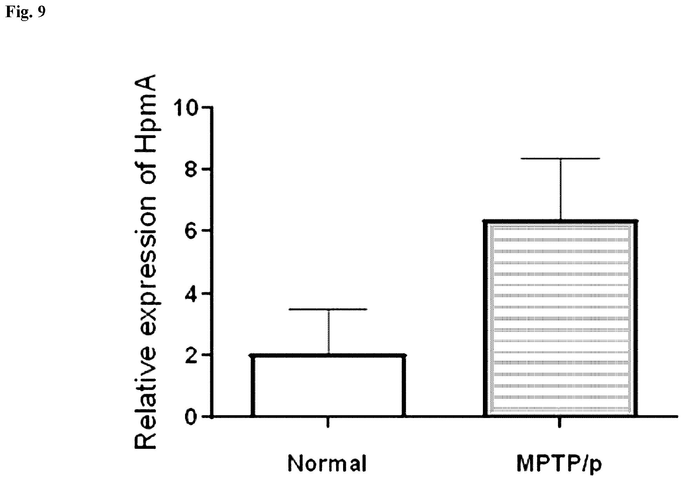

An experiment was performed to measure the amount of hpmA expression through real time polymerase chain reaction (real time-qPCR) using DNA extracted from the feces of mice in Example 11 above and primers prepared from the hpmA sequence, and the results are shown in Fig. 9. The corresponding result was calculated in terms of 16S and compared with the control group.

As shown in Fig. 9, it was found that the amount of hpmA expression in the feces of MPTP/p-induced Parkinson's disease mice was more than three times (312.14±95.84 %) compared to that of the control group (100±61.78 %). Therefore, it can be seen that hpmA has a direct relationship with Parkinson's disease.

## Claims

1. A composition for the diagnosis of a degenerative brain disease, comprising hpmA.

2. The composition according to claim 1, wherein the degenerative brain disease is Alzheimer's disease, Parkinson's disease, dementia, multiple sclerosis, Huntington's disease, amyotrophic lateral sclerosis, brain neuritis (neuroinflammation), multiple system atrophy, or Pick's disease.

3. The composition according to claim 1, wherein the composition is for the diagnosis of Parkinson's disease.

4. The composition according to claim 1, wherein the hpmA is derived from Proteus, Shigella, Klebsiella pneumoniae, or Citrobacter.

5. The composition according to claim 1, wherein the hpmA is derived from a *Proteus mirabilis* strain.

6. A method for the diagnosis of a degenerative brain disease, comprising the step of measuring the amount of hpmA from a biological sample of a subject.

7. The method according to claim 6, wherein the method further comprises the step of comparing the amount of hpmA measured from a biological sample of a subject with the amount of hpmA measured from a biological sample of a normal control group.

8. The method according to claim 7, wherein the method further comprises the step of classifying the subject as having a disease or having a high possibility of developing a disease when the amount of hpmA measured from a biological sample of a subject is greater than the amount of hpmA measured from a biological sample of a normal control group.

9. The method according to claim 6, wherein the degenerative brain disease is Alzheimer's disease, Parkinson's disease, dementia, multiple sclerosis, Huntington's disease, amyotrophic lateral sclerosis, brain neuritis (neuroinflammation), multiple system atrophy, or Pick's disease.

10. The method according to claim 6, wherein the method is for the diagnosis of Parkinson's disease.

11. The method according to claim 6, wherein the hpmAis derived from Proteus, Shigella, Klebsiella pneumoniae, or Citrobacter.

12. The method according to claim 6, wherein the hpmA is derived from a *Proteus mirabilis* strain.

13. The method according to claim 6, wherein the biological sample is tissue, blood, whole blood, serum, plasma, saliva, sputum, cerebrospinal fluid, urine, large intestine tissue or feces.

14. The method according to claim 7, wherein the normal control group is an animal, including a human, not having a degenerative brain disease.

15. A composition for the preparation of an animal model of a degenerative brain disease, comprising a strain comprising an hpmA gene as an active ingredient.

16. The composition for the preparation of an animal model of a degenerative brain disease according to claim 15, wherein the composition is a feed composition.

17. The composition for the preparation of an animal model of a degenerative brain disease according to claim 15, wherein the strain is *E. coli.*

18. A method for the preparation of an animal model of a degenerative brain disease, comprising the step of administering a strain comprising an hpmAgene to an animal excluding a human.

19. An animal model of a degenerative brain disease prepared by the method according to claim 18.

20. A method for screening a therapeutic agent for a degenerative brain disease, comprising:
(a) the step of administering a candidate drug;
(b) the step of measuring the amount of hpmA from a biological sample of a subject;
and
(c) the step of determining the therapeutic effect of a candidate drug.

21. The method for screening a therapeutic agent for a degenerative brain disease according to claim 20, wherein the method further comprises the step of measuring and comparing the amounts of hpmA before and after administration of the candidate drug.
